# EUROPEAN PATENT APPLICATION

(11) **EP 3 530 277 A1**
(43) Date of publication of application: **28.08.2019**
(21) Application number: 17826780.3
(22) Date of filing: 06.03.2017
(51) Int. Cl.: A61K 36/899, A23L 33/00, A61P 3/04, A61P 3/06, A61P 9/12, A61P 19/06, A61P 43/00, A61K 31/7016, A61K 31/702, A61K 31/205

(54) **CHINESE MEDICINE HEALTHCARE PREPARATIONS FOR SLIMMING AND WEIGHT LOSS**

(30) Priority: 15.07.2016 CN 201610560176
(71) Applicant: Gansu Dazheng Health Management Co., Ltd., Gansu Province (CN)
(72) Inventor: JIA, Xiaorong, Lanzhou Gansu 730050 (CN); WANG, Xinben, Lanzhou Gansu 730050 (CN); LI, Xia, Lanzhou Gansu 730050 (CN); LI, Wei, Lanzhou Gansu 730050 (CN); YANG, Quanlong, Gansu 730050 (CN); LIU, Shuangyan, Lanzhou Gansu 730050 (CN); YUE, Yongdong, Lanzhou Gansu 730050 (CN)
(74) Representative: ZHAOffice SPRL
(86) International application number: PCT/CN2017/075794
(87) International publication number: WO 2018/010441

(57) **Abstract**

Provided are a series of traditional Chinese medicine healthcare preparations for fat elimination and weight reduction, which are traditional Chinese medicine healthcare preparations produced by adding an appropriate raw medicine to basic active pharmaceutical ingredients consisting of rose flower, soybean protein isolate, konjac flour, soybean oligosaccharide, oat, Chinese date, and L-carnitine, or consisting of gardenia, soybean oligosaccharide, L-carnitine, and konjac flour.

## Description

### BACKGROUND

### Technical Field

The present invention relates to the field of traditional Chinese medicine healthcare products, and particularly to a traditional Chinese medicine healthcare preparation for fat elimination and weight reduction.

### Related Art

Local obesity, overweight and obesity have almost become a breeding ground for all sub-healthy status and some diseases. It is the most serious incident that plagues the health and external appearance of modern people. At present, there are many kinds of slimming products available in the market, but most of them are artificially synthesized chemical substances. Long-term use of these products can cause endocrine dysfunction, and damages to the heart, liver, kidney and other organs, cause melasma, acnes and other aesthetically unpleasant skin diseases, and even induce cancers. These products achieve the weight loss effect by suppressing the appetite of the obese patient in a short period of time by chemical drugs. It is easy to rebound after the diet returns to normal after withdrawal. Therefore, it is a general trend to research and develop a green weight-reducing product that matches the pathology of different types of obesity, has gradually exerted and stable effect, is harmless to human body, and can effect a permanent cure and also a temporary treatment.

### SUMMARY

An object of the present invention is to provide a series of traditional Chinese medicine healthcare preparations for fat elimination and weight reduction according to the pathologies of patients having different types of obesity and different body constitutions in different seasons, on the basis of syndrome differentiation and treatment of traditional Chinese medicine.

### (I) Pharmaceutical compositions of traditional Chinese medicine healthcare preparations for fat elimination and weight reduction

There are many classification methods for obesity, but if classified according to the traditional Chinese medical science, it can be divided into four types: obesity complicated by high blood pressure, high blood fat, high blood glucose, and hyperuricemia, obesity caused by too much water intake, obesity caused by too much food intake, and generalized type of obesity. Such syndrome differentiation and treatment are practically proved to be more effective. One of the fundamental causes of obesity and local obesity is the imbalance of Yin and Yang in the human body, but it varies from type to type. Therefore, balancing Yin and Yang to adjust the conversion of fat in the human body can not only achieve the effect of fat-eliminating and weight reduction, but also achieve the effect of health preserving and conditioning. The studies show that another fundamental cause of obesity and local obesity is that the human body does not compatible with and is not adapted to the characteristics of the four seasons in nature, and varies with the different seasons of spring, summer, autumn and winter. Therefore, on the basis of adjusting the human body to be harmony with the four seasons and adjusting the interior and thriving the exterior, compatible fat regulation and health preservation are carried out according to the change of four seasons in nature, which have a good effect on the fat-eliminating and weight reduction.

### 1. Fat-eliminating and weight reducing healthcare formulations for patients with different types of obesity

There are many classification methods for obesity, but if classified according to the traditional Chinese medical science, the local obesity, overweight and obesity can be divided into four types: obesity complicated by high blood pressure, high blood fat, high blood glucose, and hyperuricemia, obesity caused by too much water intake, obesity caused by too much food intake, and generalized type of obesity. Because of the different pathologies of different types of obesity, there are differences in the methods of syndrome differentiation and treatment. In addition, after long-term research, it is believed that obesity can be essentially attributed to the deficiency of spleen Yin, and overspread noxious dampness. However, obesity varies from type to type, so on the basis of tonifying spleen Yin and removing noxious dampness, four sets of fat-eliminating and weight reducing healthcare products suitable for four different type of obesity are created according to the respective characteristics of different types of obesity. The fat-eliminating and weight reducing healthcare product comprises rose flower, soybean protein isolate, konjac flour, soybean oligosaccharide, oat, Chinese date, and L-carnitine as basic active pharmaceutical ingredients, which are combined with other different active pharmaceutical ingredients, to obtain fat-eliminating and weight reducing healthcare preparations suitable for patients with different types of obesity.

### 1. Obesity complicated by high blood pressure, high blood fat, high blood glucose, and hyperuricemia

Obesity complicated by high blood pressure, high blood fat, high blood glucose, and hyperuricemia, namely, local obesity, overweight and obesity that is complicated by high blood pressure, high blood fat, high blood glucose, and hyperuricemia, and basically determined to be congenitally deficient in natural endowment, and lack of acquired nourishment. For this type of obesity, rose flower, soybean protein isolate, konjac flour, soybean oligosaccharide, oat, black soybean, Chinese date, Chinese yam, and L-carnitine are used as active pharmaceutical ingredients in the present invention. The black soybean is monarch drug, which is sweet and neutral in nature, and attributive to spleen and kidney meridians; can invigorate the circulation of blood, induce urination, dispel pathogenic wind, and detoxicate; and thus is particularly used to relieve the obesity caused by excess buildup of fat and metabolic syndrome associated therewith. Chinese date and Chinese yam are both ministerial drugs. Chinese date is sweet and mild in nature, and can replenish qi and blood. Chinese yam is sweet and neutral in nature and can promote the secretion of saliva or body fluid and dredge and adjust the upper, middle, and lower energizers. They both focus on the middle energizer spleen and stomach, and mainly act to strengthen "the postnatal foundation". Rose flower, L-carnitine, and oat are all adjuvants, can promote the circulation of qi and invigorate the circulation of blood, convert the fat and limit the absorption, and serve to effect a permanent cure. Soybean protein isolate, konjac flour, and soybean oligosaccharide are all envoy drugs, which enhance the protein supply, regulate the acidity and alkalinity of the body constitution, reconcile spleen and stomach with various medicines, balance the nutrition, and allow the monarch and ministerial drugs and adjuvants to become more reasonable and effective. All the substances cooperate with each other to coordinate the ingestion, regulate the absorption, strengthen the spleen and stomach, nourish Yin and reduce pathogenic fire, strengthen vital qi to eliminate pathogenic factors, and convert and burn the fat, to achieve the function of fat-eliminating and weight reduction, thereby comprehensively alleviating, improving and gradually remove the problem of local obesity, overweight and obesity.

The raw materials are mixed in the following parts by weight: black soybean 28-35 parts, Chinese yam 12-15 parts, Chinese date 12-15 parts, rose flower 8-10 parts, L-carnitine 8-10 parts, oat 8-10 parts, soybean protein isolate 5-6 parts, konjac flour 5-6 parts, and soybean oligosaccharide 4-6 parts.

Preferably, the raw materials are mixed in the following parts by weight: black soybean 30 parts, Chinese yam 13 parts, Chinese date 13 parts, rose flower 10 parts, L-carnitine 10 parts, oat 10 parts, soybean protein isolate 6 parts, konjac flour 6 parts, and soybean oligosaccharide 5 parts.

### 2. Obesity caused by too much water intake

Obesity caused by too much water intake is a type of obesity in which even drinking water can lead to the formation of fat, that is, obesity caused by drinking water for self rescue from the pathogenic fire resulting from chronically irregular emotin and the internal injury due to seven emotions including happy, anger, worried, to think, sadness, to fear, to be startled. For this type of obesity, rose flower, soybean protein isolate, konjac flour, soybean oligosaccharide, corn starch, oat, mulberry leaves, Chinese date, gardenia, and L-carnitine are used as active pharmaceutical ingredients in the present invention. The mulberry leaves are monarch drug, which are sweet, bitter, and cold in nature, and attributive to lung and liver meridians, can clear away lung-heat and moisturize dryness, and mainly serve to remove deficient heat in upper energizer. Chinese date and gardenia are both ministerial drugs. Chinese date is sweet and mild in nature, and can replenish qi and blood. Gardenia is bitter and cold in nature and attributive to heart, lung, and triple energizer meridians, and can clear pathogenic fire, relieve restlessness, clear away heat, promote diuresis, cool the blood, and detoxicate. They both act in the aspect of deficiency and excess. Rose flower, L-carnitine, and oat are all adjuvants, can promote the circulation of qi, invigorate the circulation of blood, convert the fat, and limit the absorption, and serve to effect a permanent cure. Soybean protein isolate, konjac flour, soybean oligosaccharide, and corn starch are all envoy drugs, which enhance the protein supply, regulate the acidity and alkalinity of the body constitution, reconcile spleen and stomach with various medicines, balance the nutrition, and allow the monarch and ministerial drugs and adjuvants to become more reasonable and effective. All the substances cooperate with each other to coordinate the ingestion, regulate the absorption, strengthen the spleen and stomach, nourish Yin and reduce pathogenic fire, strengthen vital qi to eliminate pathogenic factors, convert and burn the fat, to achieve the function of fat-eliminating and weight reduction, thereby comprehensively alleviating, improving and gradually remove the problem of local obesity, overweight and obesity.

The raw materials are mixed in the following parts by weight: mulberry leaves 22-28 parts, Chinese date 12-15 parts, gardenia 12-15 parts, rose flower 8-10 parts, L-carnitine 8-10 parts, oat 7-10 parts, soybean protein isolate 4-6 parts, konjac flour 4-6 parts, soybean oligosaccharide 4-6 parts, and corn starch 4-6 parts.

Preferably, the raw materials are mixed in the following parts by weight: mulberry leaves 25 parts, Chinese date 13 parts, gardenia 13 parts, rose flower 10 parts, L-carnitine 10 parts, oat 9 parts, soybean protein isolate 5 parts, konjac flour 5 parts, soybean oligosaccharide 5 parts, and corn starch 5 parts.

### 3. Obesity caused by too much food intake

Obesity caused by too much food intake, that is, obesity occurring in those who are over-eating, or those who have simple local hypertrophy of stomach, because of uncontrolled appetite, fatty diet, eating disorders, and injured spleen and stomach. For this type of obesity, rose flower, soybean protein isolate, konjac flour, soybean oligosaccharide, oat, Lycium chinense, Chinese date, Polygonatum sibiricum, pearl barley, semen phaseoli, and L-carnitine are used as active pharmaceutical ingredients in the present invention. Polygonatum sibiricum is monarch drug, which is sweet and neutral in nature and attributive to spleen, lung, and kidney meridians, can replenish qi, nourish Yin, invigorate the spleen, moisten the lung, and tonify the kidney, and mainly serve to condition the middle energizer spleen and stomach. Chinese date, Lycium chinense, pearl barley, and semen phaseoli are ministerial drugs. Chinese date, and Lycium chinense can nourish Yin. Pearl barley, and semen phaseoli can invigorate the spleen and remove noxious dampness. They can effect a permanent cure and also a temporary treatment, and aim at enhancing the function of the monarch drug. Rose flower, L-carnitine, and oat are all adjuvants, can promote the circulation of qi, invigorate the circulation of blood, convert the fat, and limit the absorption, and serve to effect a permanent cure. Soybean protein isolate, konjac flour, and soybean oligosaccharide are all envoy drugs, which enhance the protein supply, regulate the acidity and alkalinity of the body constitution, and reconcile spleen and stomach with various medicines, and allow the monarch and ministerial drugs and adjuvants to become more reasonable and effective. All the substances cooperate with each other to coordinate the ingestion, regulate the absorption, strengthen the spleen and stomach, nourish Yin and reduce pathogenic fire, strengthen vital qi to eliminate pathogenic factors, and convert and burn the fat, to achieve the function of fat-eliminating and weight reduction, thereby comprehensively alleviating, improving and gradually remove the problem of local obesity, overweight and obesity.

The raw materials are mixed in the following parts by weight: Polygonatum sibiricum 20-25 parts, Chinese date 9-12 parts, Lycium chinense 9-12 parts, pearl barley 9-12 parts, semen phaseoli 9-12 parts, rose flower 7-8 parts, oat 7-8 parts, L-carnitine 7-8 parts, soybean protein isolate 2-4 parts, konjac flour 2-4 parts, and soybean oligosaccharide 2-4 parts.

Preferably, the raw materials are mixed in the following parts by weight: Polygonatum sibiricum 23 parts, Chinese date 11 parts, Lycium chinense 11 parts, pearl barley 11 parts, semen phaseoli 11 parts, rose flower 8 parts, oat 8 parts, L-carnitine 8 parts, soybean protein isolate 3 parts, konjac flour 3 parts, and soybean oligosaccharide 3 parts.

### 4. Generalized type of obesity obesity

Generalized type of obesityobesity, that is, generalized type of obesity that is caused by excess mental work, physical work, and sexual activity, and cannot be classified into the above three groups. For this type of obesity, rose flower, soybean protein isolate, konjac flour, soybean oligosaccharide, oat, Lycium chinense, Chinese date, peach kernel, dried persimmon, laminalia, herba lophatheri, gardenia, Polygonatum sibiricum, pearl barley, mulberry leaves, and L-carnitine are used as active pharmaceutical ingredients in the present invention. Lycium chinense is monarch drug, which is sweet and neutral in nature and attributive to liver and kidney meridians, can nourish the liver and kidney and replenish the vital essence to improve eyesight, and is used to alleviate consumptive disease, deficient vital essence, soreness of waist and knee joint, dizziness and tinnitus, and internal heat dispersion-thirst, to endeavor to restore the foundation. Chinese date, and peach kernel are ministerial drugs. Chinese date has the function of tonification, because long-term overweight will necessarily lead to weakness. Peach kernel dissolves the stagnant, because long-term overweight will necessarily lead to stagnant, and effects a permanent cure and also a temporary treatment. Rose flower, oat, L-carnitine, dried persimmon, laminalia, herba lophatheri, gardenia, Polygonatum sibiricum, pearl barley, and mulberry leaves are all adjuvants, which work together to deal with the complicated status of the difficult-to-be-categorized obesity by promoting the circulation of qi, invigorating the circulation of blood, softening and resolving hard mass, strengthening vital qi to eliminate pathogenic factors, and removing dampness and eliminating fat. Soybean protein isolate, konjac flour, and soybean oligosaccharide are all envoy drugs, which enhance the protein supply, regulate the acidity and alkalinity of the body constitution, and reconcile spleen and stomach with various medicines, and allow the monarch and ministerial drugs and adjuvants to become more reasonable and effective. All the substances cooperate with each other to coordinate the ingestion, regulate the absorption, strengthen the spleen and stomach, nourish Yin and reduce pathogenic fire, strengthen vital qi to eliminate pathogenic factors, and convert and burn the fat, to achieve the function of fat-eliminating and weight reduction, thereby comprehensively alleviating, improving and gradually remove the problem of local obesity, overweight and obesity.

The raw materials are mixed in the following parts by weight: Lycium chinense 18-24 parts, Chinese date 8-12 parts, peach kernel 8-12 parts, rose flower 4-6 parts, oat 4-6 parts, L-carnitine 4-6 parts, dried persimmon 4-6 parts, laminalia 4-6 parts, herba lophatheri 4-6 parts, gardenia 4-6 parts, Polygonatum sibiricum 4-6 parts, pearl barley 4-6 parts, mulberry leaves 4-6 parts, soybean protein isolate 3-4 parts, konjac flour 2-3 parts, and soybean oligosaccharide 2-3 parts.

Preferably, the raw materials are mixed in the following parts by weight: Lycium chinense 20 parts, Chinese date 10 parts, peach kernel 10 parts, rose flower 5 parts, oat 5 parts, L-carnitine 5 parts, dried persimmon 5 parts, laminalia 5 parts, herba lophatheri 5 parts, gardenia 5 parts, Polygonatum sibiricum 5 parts, pearl barley 5 parts, mulberry leaves 5 parts, soybean protein isolate 4 parts, konjac flour 3 parts, and soybean oligosaccharide 3 parts.

### II. Fat-eliminating and weight reducing healthcare preparations for "Yin obesity", "Yang obesity"

Based on the ideals of traditional Chinese medical science, local obesity and obesity are classified into two types: "Yin obesity" and "Yang obesity" according to the theory of "Yin and Yang" in traditional Chinese medical science. The type of obesity caused by the spread of Yin coldness, Yin dampness, and Yin toxicity in the body is defined as "Yin" obesity, and the pale tongue coating is used as the determination criterion. The type of obesity caused by the spread of Yang heat, Yang dryness and Yang toxicity in the body is defined as "Yang" obesity, and the yellowish tongue coating is used as the determination criterion. Because of the different pathologies of different types of obesity, the methods of syndrome differentiation and treatment are different. Based on the formulation disclosed in Patent Application No. 201610560176.1, the conversion of the body fat is promoted and thus the functions of slimming, health invigorating, charm increasing and health preserving are achieved by syndrome differentiation and treatment comprising balancing the Yin and Yang, strengthening vital qi to eliminate pathogenic factors, and invigorating the spleen and stomach according to the features of "Yin obesity" and "Yang obesity" in the present invention, thereby comprehensively alleviating, improving and gradually eliminating the problems of local obesity, overweight and obesity.

### 1. Fat-eliminating and health-preserving preparations for "Yin obesity"

Pearl barley, rose flower, soybean protein isolate, konjac flour, soybean oligosaccharide, oat, Chinese date, and L-carnitine are used as raw materials. The mechanism of combination is as follows. Pearl barley is monarch drug, which is sweet, flat, and cool in nature and attributive to spleen, stomach, and lung meridians, and can eliminate the Yin coldness, Yin dampness, and Yin toxicity in the body to manage the "fat" globally, by invigorating the spleen, excreting the dampness, dispelling the stasis, stopping diarrhea, clearing the heat, and discharging the pus. Chinese date is ministerial drug, which is sweet and mild, replenishes qi and blood, focuses on the middle energizer spleen and stomach, takes qi and blood as a lead, mainly serves to strengthen "the postnatal foundation", and improves various physiological functions of human as well. Rose flower, L-carnitine, and oat are all adjuvants, which promote the circulation of qi, invigorate the circulation of blood, convert the fat, limit the absorption, and serve to effect a permanent cure. Soybean protein isolate, konjac flour, and soybean oligosaccharide are all envoy drugs, which enhance the protein supply, regulate the acidity and alkalinity of the body constitution, and reconcile spleen and stomach with various medicines, and allow the monarch and ministerial drugs and adjuvants to become more reasonable and effective. The whole formulation can not only balance Yin and Yang and manage the fat, but also preserve the health and wellness.

The raw materials are mixed in the following parts by weight: pearl barley 30-35 parts, Chinese date 18-22 parts, rose flower 8-12 parts, L-carnitine 8-12 parts, oat 8-12 parts, soybean protein isolate 5-7 parts, konjac flour 5-7 parts, and soybean oligosaccharide 4-6 parts.

Preferably, the raw materials are mixed in the following parts by weight: pearl barley 33 parts, Chinese date 20 parts, rose flower 10 parts, L-carnitine 10 parts, oat 10 parts, soybean protein isolate 6 parts, konjac flour 6 parts, and soybean oligosaccharide 5 parts.

### 2. Fat-eliminating and health-preserving preparations for "Yang obesity"

Red bean, corn starch, rose flower, soybean protein isolate, konjac flour, soybean oligosaccharide, oat, Chinese date, and L-carnitine are used as raw materials. The mechanism of combination is as follows. Red bean is monarch drug, which is sweet, acidic, and neutral in nature and is attributive to heart and small intestine meridians, and can eliminating the Yang heat, Yang dryness and Yang toxicity in the body by inducing urination, diminishing swelling, detoxicating, and discharging gus, to manage the "fat" globally. Chinese date is ministerial drug, which is sweet and mild, replenishes qi and blood, focuses on the middle energizer spleen and stomach and qi and blood in the whole body, mainly serves to strengthen "the postnatal foundation", and improves various physiological functions of human as well. Rose flower, L-carnitine, and oat are all adjuvants, which can promote the circulation of qi, invigorate the circulation of blood, convert the fat, limit the absorption, and serve to effect a permanent cure. Soybean protein isolate, konjac flour, soybean oligosaccharide, and corn starch are all envoy drugs, which enhance the protein supply, regulate the acidity and alkalinity of the body constitution, reduce the intake of heat, and reconcile spleen and stomach with various medicines, and allow the monarch and ministerial drugs and adjuvants to become more reasonable and effective. The whole formulation can not only balance Yin and Yang and manage the fat, but also preserve the health and wellness.

The raw materials are mixed in the following parts by weight: red bean 28-32 parts, Chinese date 18-22 parts, rose flower 8-12 parts, L-carnitine 8-12 parts, oat 8-12 parts, soybean protein isolate 5-7 parts, konjac flour 5-7 parts, soybean oligosaccharide 5-7 parts, and corn starch 5-7 parts.

Preferably, the raw materials are mixed in the following parts by weight: red bean 30 parts, Chinese date 20 parts, rose flower 10 parts, L-carnitine 10 parts, oat 10 parts, soybean protein isolate 5 parts, konjac flour 5 parts, soybean oligosaccharide 5 parts, and corn starch 5 parts.

### III. Fat-eliminating and weight reducing healthcare preparations for different seasons

Based on the syndrome differentiation and treatment, the fat-eliminating and weight-reducing health preservation is combined with the seasonal change, according to the health-preservation theory of "germination in spring", "growth in summer", "harvest in autumn", and "hiding in winter". Scientific combination is attained using raw materials of medicines and foods having the same origin, and the excess fat of the human body is converted by regulating different nutrient intake and absorption in different seasons, and by balancing Yin and Yang, strengthening vital qi to eliminate pathogenic factors, and invigorating the spleen and stomach, to achieve the functions of slimming, health invigorating, charm increasing and health preserving, thereby comprehensively alleviating, and gradually improving the problem of local obesity, overweight and obesity, and various sub-healthy states.

### 1. Fat-eliminating and health-preserving healthcare preparation for consumption in spring

The health-preserving healthcare preparation for management of fat in spring according to the present invention comprises a combination of fried licorice root, Chinese date, gardenia, soybean oligosaccharide, L-carnitine, and konjac flour. The mechanism of combination is as follows. Fried licorice root is monarch drug, which is sweet and neutral in nature and attributive to heart, lung, spleen, and stomach meridians, and can promote "germination in spring" fully and manage the "fat" globally by the functions of tonifying spleen and supplementing the vital energy to adapt to the seasonal characteristics that "germination dominates in spring". Chinese date is ministerial drug, which is sweet and mild in nature, replenishes qi and blood, focuses on the middle energizer spleen and stomach, takes qi and blood as a lead, mainly serves to strengthen "the postnatal foundation", and improves various physiological functions of human as well. Gardenia is an adjuvant, which is bitter and cold in nature and attributive to heart, lung, and triple energizer meridians, can convert the fat and limit the absorption by clearing pathogenic fire, relieving restlessness, clearing away heat and promoting diuresis, cooling the blood, and detoxicating, and serve to effect a permanent cure. Soybean oligosaccharide, L-carnitine, and konjac flour are all envoy drugs, which can balance the nutrient supply, regulate the acidity and alkalinity of the body constitution, and allow the monarch and ministerial drugs and adjuvants to become more reasonable and effective. The whole formulation can not only provide a good solution for "nourishment in spring", but also manage the fat and preserve the health and wellness.

To achieve a desirable health preservation effect, the raw materials are mixed in the following parts by weight: fried licorice root 32-38 parts, Chinese date 19-22 parts, gardenia 14-18 parts, soybean oligosaccharide 6-10 parts, L-carnitine 6-10 parts, and konjac flour 6-10 parts.

Preferably, the raw materials are mixed in the following parts by weight: fried licorice root 35 parts, Chinese date 20 parts, gardenia 15 parts, soybean oligosaccharide 10 parts, L-carnitine 10 parts, and konjac flour 10 parts.

### 2. Fat-eliminating and health-preserving healthcare preparation for consumption in summer

The health-preserving healthcare preparation for management of fat in summer according to the present invention comprises a combination of herba lophatheri, semen phaseoli, Lycium chinense, gardenia, soybean oligosaccharide, L-carnitine, and konjac flour. The mechanism of combination is as follows. Herba lophatheri is monarch drug, which is sweet, flat, and cold in nature and attributive to heart, stomach, and small intestine meridians, and can promote "growth in summer" fully and manage the fat globally by the functions of clearing away heat, relieving restlessness, and promoting diuresis to adapt to the seasonal characteristics that "growth dominates in summer". Semen phaseoli is ministerial drug, which is sweet, sour and neutral in nature and attributive to heart and small intestine meridians, can induce urination, diminish swelling, detoxicate and discharge gus, and serves to dispel noxious dampness primarily and to enhance the function of clearing away noxious dampness mainly by small intestine. Gardenia and Lycium chinense are both adjuvants. Gardenia is bitter and cold in nature and attributive to heart, lung, and triple energizer meridians, can convert the fat and limit the absorption by clearing away pathogenic fire, relieving restlessness, clearing away heat, promoting diuresis, cooling the blood, and detoxicating, and focuses on expelling pathogens, thus effecting a permanent cure. Lycium chinense is sweet and neutral in nature and attributive to liver and kidney meridians, can nourish the liver and kidney and replenish the vital essence to improve eyesight, endeavors to restore the foundation, and serves to effect a permanent cure. Soybean oligosaccharide, L-carnitine, and konjac flour are all envoy drugs, which can balance the nutrient supply, regulate the acidity and alkalinity of the body constitution, and allow the monarch and ministerial drugs and adjuvants to become more reasonable and effective. The whole formulation can not only provide a good solution for "nourishment in summer", but also manage the fat and preserve the health and wellness.

To achieve a desirable health preservation effect, the raw materials are mixed in the following parts by weight: herba lophatheri 28-32 parts, semen phaseoli 20-24 parts, Lycium chinense 14-17 parts, gardenia 14-18 parts, soybean oligosaccharide 6-10 parts, L-carnitine 6-10 parts, and konjac flour 6-10 parts.

Preferably, the raw materials are mixed in the following parts by weight: herba lophatheri 30 parts, semen phaseoli 22 parts, Lycium chinense 15 parts, gardenia 15 parts, soybean oligosaccharide 6 parts, L-carnitine 6 parts, and konjac flour 6 parts.

### 3. Fat-eliminating and health-preserving healthcare preparation for consumption in autumn

The health-preserving healthcare preparation for management of fat in autumn according to the present invention comprises a combination of Chinese yam, Polygonatum sibiricum, Lycium chinense, gardenia, soybean oligosaccharide, L-carnitine, and konjac flour as raw materials. The mechanism of combination is as follows. Chinese yam is monarch drug, which is sweet and neutral in nature and attributive to spleen, lung, and kidney meridians, and can promote "harvest in autumn" fully and manage the fat globally by the functions of tonifying spleen, nourishing stomach, promoting the secretion of saliva or body fluid, invigorating lung, tonifying kidney, and arresting seminal emission to adapt to the seasonal characteristics that "harvest dominates in autumn". Polygonatum sibiricum is ministerial drug, which is sweet and neutral in nature and attributive to spleen, lung, and kidney meridians, can replenish qi, nourish Yin, invigorate the spleen, moisten the lung, and tonify the kidney, focuses on dredging and nourishing the upper, middle and lower energizers, and mainly serves to enhance the healthy qi in human body and improve various physiological functions of human. Gardenia and Lycium chinense are both adjuvants. Gardenia is bitter and cold in nature and attributive to heart, lung, and triple energizer meridians, can convert the fat and limit the absorption by clearing pathogenic fire, relieving restlessness, clearing away heat, promoting diuresis, cooling the blood, and detoxicating, and focuses on expelling pathogens, thus effecting a permanent cure. Lycium chinense is sweet and neutral in nature and attributive to liver and kidney meridians, can nourish the liver and kidney and replenish the vital essence to improve eyesight, endeavors to restore the foundation, and serves to effect a permanent cure. Soybean oligosaccharide, L-carnitine, and konjac flour are all envoy drugs, which can balance the nutrient supply, regulate the acidity and alkalinity of the body constitution, and allow the monarch and ministerial drugs and adjuvants to become more reasonable and effective. The whole formulation can not only provide a good solution for "nourishment in autumn", but also manage the fat and preserve the health and wellness.

To achieve a desirable health preservation effect, the raw materials are mixed in the following parts by weight: Chinese yam 28-32 parts, Polygonatum sibiricum 20-24 parts, Lycium chinense 14-17 parts, gardenia 14-18 parts, soybean oligosaccharide 6-10 parts, L-carnitine 6-10 parts, and konjac flour 6-10 parts.

Preferably, the raw materials are mixed in the following parts by weight: Chinese yam 30 parts, Polygonatum sibiricum 22 parts, Lycium chinense 15 parts, gardenia 15 parts, soybean oligosaccharide 6 parts, L-carnitine 6 parts, and konjac flour 6 parts.

### 4. Fat-eliminating and health-preserving healthcare preparation for consumption in winter

The health-preserving healthcare preparation for management of fat in winter according to the present invention comprises a combination of peach kernel, laminalia, gardenia, soybean oligosaccharide, L-carnitine, and konjac flour as raw materials. The mechanism of combination is as follows. Peach kernel is monarch drug, which is bitter, sweet, and neutral in nature and attributive to heart, liver, and large intestine meridians, and can promote "hiding in winter" fully and manage the fat globally by the functions of invigorating the circulation of blood, dissolving the stagnant, and loosening the bowels to relieve constipation to adapt to the seasonal characteristics that "hiding dominates in winter". Laminalia is ministerial drug, which is salty and cold in nature and attributive to liver, stomach, and kidney meridians, can soften and resolve hard mass, reduce phlegm, and induce urination, and can dissolve the fat and remove evil pathogens in combination with the monarch drug. Gardenia is adjuvant, which is bitter and cold in nature and attributive to heart, lung, and triple energizer meridians, can convert the fat and limit the absorption by clearing pathogenic fire, relieving restlessness, clearing away heat and promoting diuresis, cooling the blood, and detoxicating, focuses on removing pathogens, and serves to effect a permanent cure. Soybean oligosaccharide, L-carnitine, and konjac flour are all envoy drugs, which can balance the nutrient supply, regulate the acidity and alkalinity of the body constitution, and allow the monarch and ministerial drugs and adjuvants to become more reasonable and effective. The whole formulation can not only provide a good solution for "nourishment in winter", but also manage the fat and preserve the health and wellness.

To achieve a desirable health preservation effect, the raw materials are mixed in the following parts by weight: peach kernel 33-37 parts, laminalia 20-23 parts, gardenia 14-18 parts, soybean oligosaccharide 6-10 parts, L-carnitine 6-10 parts, and konjac flour 6-10 parts.

Preferably, the raw materials are mixed in the following parts by weight: peach kernel 35 parts, laminalia 22 parts, gardenia 15 parts, soybean oligosaccharide 10 parts, L-carnitine 10 parts, and konjac flour 10 parts.

The traditional Chinese medicine healthcare preparation for fat elimination and weight reduction can be prepared into any kind of orally taken preparations following a pharmaceutically conventional process, such as tablets, granules, pills, tea preparations, and capsules, and preferably tea preparations and granules; or be prepared into health food and food according to a processing technology of health food and food, such as biscuits, meal replacement tea powders, solid beverage, coffee, and bread.

### (II) Clinical data

### I. Fat-eliminating and weight-reducing healthcare preparations for different types of obesity

Clinical data is provided below to illustrate the fat management effect of the health preserving and slimming preparations comprising different combinations of raw materials of the present invention for local obesity, overweight and obesity.

### 1. Objects and methods

### 1.1. Test materials

A series of tea preparation products according to the present invention, known as black soybean-Chinese date meal replacement tea powder, mulberry leaves-Chinese date meal replacement tea powder, Polygonatum sibiricum-Chinese date meal replacement tea powder, and Lycium chinense-Chinese date meal replacement tea powder; specification: 400 g/bag x2 bag/box; supplied by Lanzhou Gucci Biotechnology Co., Ltd.

### 1.2. Test objects

A total of 240 patients aged 14 to 63 years of four different types of obesity were observed in Lanzhou, and statistical calculation was made with no differentiation between male and female. There were 60 cases of obesity complicated by high blood pressure, high blood fat, high blood glucose, and hyperuricemia, 40 cases of obesity caused by too much water intake, 60 cases of obesity caused by too much food intake, and 80 cases of generalized type of obesity. The patients of different types of obesity were respectively statistically calculated based on mild obesity (where the body weight is greater than [height (cm)-100]x0.9x1.2), moderate obesity (where the body weight is greater than [height (cm)-100]x0.9x1.4), and serious obesity (where the body weight is greater than [height (cm)-100]x0.9x1.6), accompanied by a correspondingly elevated rate of body fat.

### 1.3. Method of using

Usage and dosage: once in the morning and evening, 1 scoop each time (about 40 g), can replace the main meal. During the test period, juice, sugar water, and high sugar drinks are forbidden. 1 course of treatment per month. Generally observation over 3 courses of treatment is needed.

### 2. Determination criteria for therapeutic effects

Curative: The body weight and body fat rate return to normal standards and do not rebound after half a year of follow-up. Obviously effective: 40% or more of the excess body weight and body fat rate is reduced. Effective: 20% or more of the excess body weight and body fat rate is reduced. Ineffective: less than 20% of the excess body weight and body fat rate is reduced. The body fat rate is tested using an OMRON fat tester.

### 3. Test results

Statistical calculation was performed after 90 days of treatment and observation. The results are shown in Table 1:

**Table 1. Clinical data of fat-eliminating and weight reducing effects for different types of obesity**

| Type of obesity | Case number | Curative | | Obviously effective | | Effective | | Ineffective | |
|---|---|---|---|---|---|---|---|---|---|
| | | Case number | Percentage | Case number | Percentage | Case number | Percentage | Case number | Percentage |
| Obesity complicated by high blood pressure, high blood fat, high blood glucose, and hyperuricemia | 60 | 19 | 31.68 | 17 | 28.34 | 13 | 21.18 | 11 | 18.34 |
| Obesity caused by too much water intake | 40 | 13 | 32.50 | 9 | 22.50 | 8 | 20.00 | 10 | 25.00 |
| Obesity with too much food intake | 60 | 27 | 45.00 | 13 | 21.66 | 11 | 18.34 | 9 | 15.00 |
| Generalized type of obesity | 80 | 32 | 40.00 | 14 | 17.50 | 18 | 22.50 | 16 | 20.00 |

The above clinical statistical data shows that the series of fat-eliminating and weight-reducing traditional Chinese medicine preparations of the present invention have obvious fat-eliminating and weight reducing effects for the corresponding types of obesity. The total effective rate reaches 80.84%; although the patient having ineffective treatment has little change in body weight, there are changes in measurements and appearance; and all subjects consciously feel physically healthy.

### II., Fat-eliminating and weight-reducing healthcare preparations for "Yin obesity" and "Yang obesity"

### 1. Objects and methods

### 1.1. Test materials

Meal replacement tea powders of the present invention, specification: 20 g/bag x 20 bag/box. Product name: pearl barley-Chinese date meal replacement tea powder (Yin obesity) and red bean-Chinese date meal replacement tea powder (Yang obesity), supplied by Lanzhou Gucci Biotechnology Co., Ltd.

### 1.2. Test objects

Patients with different severities of obesity were respectively statistically calculated based on mild obesity (where the body weight is greater than [height (cm)-100]x0.9x1.2), moderate obesity (where the body weight is greater than [height (cm)-100]x0.9x1.4), and serious obesity (where the body weight is greater than [height (cm)-100]x0.9x1.6), accompanied by a correspondingly elevated rate of body fat. Then, the patients with different severities of obesity were grouped into "Yin-obese" and "Yang-obese" patients according to the physical signs. There were 120 patients that were male and aged 14-63 years. Determination of "Yin-obese" and "Yang-obese" patients: the spread of Yin coldness, Yin dampness, and Yin toxicity in the body is defined as "Yin obesity", and the pale tongue coating is used as the determination criterion; and the spread of Yang heat, Yang dryness and Yang toxicity in the body is defined as "Yang obesity", and the yellowish tongue coating is used as the determination criterion.

### 1.3. Method of using

Usage and dosage: once in the morning and evening, 4 bags each time. The effect is more desirable if consumed before exercise. Oral administration with juice, sugar water, and high sugar drinks are forbidden. During the conditioning period, no fruits and snacks are allowed. 1 course of treatment per month. Generally observation over 3 courses of treatment is needed.

### 2. Determination criteria for therapeutic effects

Curative: The body weight and body fat rate return to normal standards and do not rebound after half a year of follow-up. Obviously effective: 40% or more of the excess body weight and body fat rate is reduced. Effective: 20% or more of the excess body weight and body fat rate is reduced. Ineffective: less than 20% of the excess body weight and body fat rate is reduced. The body fat rate is tested using an OMRON fat tester.

### 3. Test results

Statistical calculation was performed after 90 days of treatment and observation. The results are shown in Tables 2 and 3.

**Table 2. Clinical data of fat-eliminating and weight reducing effects of the fat-eliminating and health-preserving preparations for "Yin-obese" patients**

| Severity of obesity | Case number | Curative | | Obviously effective | | Effective | | Ineffective | |
|---|---|---|---|---|---|---|---|---|---|
| | | Case number | Percentage | Case number | Percentage | Case number | Percentage | Case number | Percentage |
| Mild obesity | 15 | 6 | 40.00 | 7 | 46.67 | 2 | 13.33 | 0 | |
| Moderate obesity | 30 | 8 | 26.67 | 13 | 43.33 | 7 | 23.33 | 2 | 6.67 |
| Serious obesity | 15 | 3 | 20.00 | 4 | 28.67 | 6 | 40.00 | 2 | 13.33 |

**Table 3. Clinical data of fat-eliminating and weight reducing effects of the fat-eliminating and health-preserving preparations for "Yang-obese" patients**

| Severity of obesity | Case number | Curative | | Obviously effective | | Effective | | Ineffective | |
|---|---|---|---|---|---|---|---|---|---|
| | | Case number | Percentage | Case number | Percentage | Case number | Percentage | Case number | Percentage |
| Mild obesity | 18 | 4 | 22.22 | 7 | 38.89 | 6 | 33.33 | 1 | 5.56 |
| Moderate obesity | 20 | 6 | 30.00 | 7 | 25.00 | 4 | 20.00 | 3 | 15.00 |
| Serious obesity | 22 | 4 | 18.18 | 8 | 36.36 | 7 | 31.82 | 3 | 13.64 |

The above clinical statistical results show that the fat-eliminating and weight-reducing preparations of the present invention have a total effective rate of 93.33% for "Yin obesity", and a total effective rate of 88.33% for "Yang obesity". Moreover, in the present invention, the pure natural raw materials of medicines and foods having the same origin are used as active pharmaceutical ingredients, which have the health-preserving and healthcare effects while achieving the effect of fat elimination and weight reduction.

### III. Fat-eliminating and weight-reducing healthcare preparations for different seasons

### 1. Objects and methods

### 1.1. Test materials

Meal replacement tea powders, specification: 20 g/bag x 20 bags/box. Product name: dedicated meal replacement tea powder of fried licorice root (spring), dedicated meal replacement tea powder of herba lophatheri (summer), dedicated meal replacement tea powder of Chinese yam (autumn), and dedicated meal replacement tea powder of peach kernel (winter), supplied by Lanzhou Gucci Biotechnology Co., Ltd.

### 1.2. Test objects

A total of 360 patients aged 14 to 63 years of four different types of obesity were observed in Lanzhou, and statistical calculation was made with no differentiation between male and female. The patients of different types of obesity were respectively statistically calculated based on m mild obesity (where the body weight is greater than [height (cm)-100]x0.9x1.2), moderate obesity (where the body weight is greater than [height (cm)-100]x0.9x1.4), and serious obesity (where the body weight is greater than [height (cm)-100]x0.9x1.6), accompanied by a correspondingly elevated rate of body fat.

### 1.3. Method of using

Usage and dosage: once in the evening, 4 bags each time, orally take corresponding products according to the seasons. The effect is more desirable if consumed before exercise. 1 course of treatment per month. Observation over 3 courses of treatment is needed.

### 2. Determination criteria for therapeutic effects

Curative: The body weight and body fat rate return to normal standards and do not rebound after half a year of follow-up. Obviously effective: 40% or more of the excess body weight and body fat rate is reduced. Effective: 20% or more of the excess body weight and body fat rate is reduced. Ineffective: less than 20% of the excess body weight and body fat rate is reduced. The body fat rate is tested using an OMRON fat tester.

### 3. Test results

Statistical calculation was performed after 90 days of treatment and observation. The results are shown in Tables 4-7.

**Table 4. Clinical data of fat-eliminating effects of dedicated meal replacement tea powder of fried licorice root (spring) for patients with various different types of obesity**

| Severity of obesity | Case number | Curative | | Obviously effective | | Effective | | Ineffective | |
|---|---|---|---|---|---|---|---|---|---|
| | | Case number | Percentage | Case number | Percentage | Case number | Percentage | Case number | Percentage |
| Mild obesity | 30 | 18 | 60 | 9 | 30 | 3 | 10 | 0 | 0 |
| Moderate obesity | 30 | 12 | 40 | 11 | 36.7 | 5 | 16.6 | 2 | 6.7 |
| Serious obesity | 30 | 6 | 20 | 8 | 26.7 | 12 | 40 | 4 | 13.3 |

**Table 5. Clinical data of fat-eliminating effects of dedicated meal replacement tea powder of herba lophatheri (summer) for patients with various different types of obesity**

| Severity of obesity | Case number | Curative | | Obviously effective | | Effective | | Ineffective | |
|---|---|---|---|---|---|---|---|---|---|
| | | Case number | Percentage | Case number | Percentage | Case number | Percentage | Case number | Percentage |
| Mild obesity | 30 | 19 | 63.4 | 7 | 23.3 | 4 | 13.3 | 0 | 0 |
| Moderate obesity | 30 | 9 | 30 | 13 | 43.3 | 7 | 23.4 | 1 | 3.3 |
| Serious obesity | 30 | 5 | 16.7 | 11 | 36.7 | 11 | 36.6 | 3 | 10 |

**Table 6. Clinical data of fat-eliminating effects of dedicated meal replacement tea powder of Chinese yam (autumn) for patients with various different types of obesity**

| Severity of obesity | Case number | Curative | | Obviously effective | | Effective | | Ineffective | |
|---|---|---|---|---|---|---|---|---|---|
| | | Case number | Percentage | Case number | Percentage | Case number | Percentage | Case number | Percentage |
| Mild obesity | 30 | 13 | 43.3 | 11 | 36.7 | 6 | 20 | 0 | 0 |
| Moderate obesity | 30 | 7 | 23.3 | 15 | 50 | 5 | 16.7 | 3 | 10 |
| Serious obesity | 30 | 5 | 16.7 | 13 | 43.3 | 7 | 23.3 | 5 | 16.7 |

**Table 7. Clinical data of fat-eliminating effects of dedicated meal replacement tea powder of peach kernel (winter) for patients with various different types of obesity**

| Severity of obesity | Case number | Curative | | Obviously effective | | Effective | | Ineffective | |
|---|---|---|---|---|---|---|---|---|---|
| | | Case number | Percentage | Case number | Percentage | Case number | Percentage | Case number | Percentage |
| Mild obesity | 30 | 8 | 26.7 | 7 | 23.3 | 13 | 43.3 | 2 | 6.7 |
| Moderate obesity | 30 | 6 | 20 | 8 | 26.7 | 12 | 40 | 4 | 13.3 |
| Serious obesity | 30 | 6 | 20 | 12 | 40 | 6 | 20 | 6 | 20 |

The above clinical statistical data shows that the series of fat-eliminating, weight-reducing health-preserving and healthcare preparations for different seasons of the present invention have obvious fat-eliminating and weight reducing effects for different types of obesity, with a total effective rate reaching 83.33%. Moreover, in the present invention, the pure natural raw materials of medicines and foods having the same origin are used as active pharmaceutical ingredients, which have the health-preserving and healthcare effects while achieving the effect of fat elimination and weight reduction.

### DETAILED DESCRIPTION

The mixing ratio of raw materials in and the preparation of the traditional Chinese medicine preparation for fat elimination and weight reduction according to the present invention are further illustrated by way of specific examples below.

### Example 1: Black soybean-Chinese date meal replacement tea powder

Mixing ratio of raw materials: black soybean 30 parts, Chinese yam 13 parts, Chinese date 13 parts, rose flower 10 parts, L-carnitine 10 parts, oat 10 parts, soybean protein isolate 6 parts, konjac flour 6 parts, and soybean oligosaccharide 5 parts.

Preparation process: The raw materials were mixed according to the above proportions, pulverized, granulated, sterilized, and packaged, to prepare a tea having a specification o 400 g/bag x 2 bags/box.

Usage and dosage: once in the morning and evening, 1 scoop each time (about 40 g), can replace the main meal. The effect is more desirable if consumed before exercise. Orally taken after being dissolved in a suitable amount of, if preferred, warm water, yogurt, milk, soy milk, milkshake, tea, vegetable juice, liquid coffee, almond milk, coconut juice, fermented glutinous rice soup, mineral water, or pure water (where the following drinks: juice, sugar water, and high sugar drinks are forbidden!). 1 course of treatment per month. Generally observation over 3 courses of treatment is needed.

Applicable to: those with local obesity, overweight, and obesity complicated by high blood pressure, high blood fat, high blood glucose, and hyperuricemia.

### Example 2: Mulberry leaves-Chinese date meal replacement tea powder

Mixing ratio of raw materials: mulberry leaves 25 parts, Chinese date 13 parts, gardenia 13 parts, rose flower 10 parts, L-carnitine 10 parts, oat 9 parts, soybean protein isolate 5 parts, konjac flour 5 parts, soybean oligosaccharide 5 parts, and corn starch 5 parts.

Preparation process: the same as that in Example 1.

Usage and dosage: the same as that in Example 1.

Applicable to: patients with obesity caused by too much water intake, that is, a type of obesity in which even drinking water can lead to the formation of fat.

### Example 3: Polygonatum sibiricum-Chinese date meal replacement tea powder

Mixing ratio of raw materials: Polygonatum sibiricum 23 parts, Chinese date 11 parts, Lycium chinense 11 parts, pearl barley 11 parts, semen phaseoli 11 parts, rose flower 8 parts, oat 8 parts, L-carnitine 8 parts, soybean protein isolate 3 parts, konjac flour 3 parts, and soybean oligosaccharide 3 parts.

Preparation process: the same as that in Example 1.

Usage and dosage: the same as that in Example 1.

Applicable to: those with obesity caused by too much food intake, or those who have simple local hypertrophy of stomach.

### Example 4: Lycium chinense-Chinese date meal replacement tea powder

Mixing ratio of raw materials: Lycium chinense 20 parts, Chinese date 10 parts, peach kernel 10 parts, rose flower 5 parts, oat 5 parts, L-carnitine 5 parts, dried persimmon 5 parts, laminalia 5 parts, herba lophatheri 5 parts, gardenia 5 parts, Polygonatum sibiricum 5 parts, pearl barley 5 parts, mulberry leaves 5 parts, soybean protein isolate 4 parts, konjac flour 3 parts, and soybean oligosaccharide 3 parts.

Preparation process: the same as that in Example 1.

Usage and dosage: the same as that in Example 1.

Applicable to: those with generalized type of obesity that cannot be classified into the above three groups.

### Example 5: Pearl barley-Chinese date meal replacement tea powder

Mixing ratio of raw materials (in parts by weight): pearl barley 33 parts, Chinese date 20 parts, rose flower 10 parts, L-carnitine 10 parts, oat 10 parts, soybean protein isolate 6 parts, konjac flour 6 parts, and soybean oligosaccharide 5 parts.

Preparation process: The raw materials were mixed according to the above proportions, pulverized, sterilized, and packaged, to prepare a tea having a specification of 20 g/bag x 20 bags/box.

Applicable to: those having local obesity or obesity complicated by pale tongue coating, and essentially determined to be "Yin-obese".

Usage and dosage: once in the morning and evening, 4 bags each time, dedicated for consumption in the morning and evening. The effect is more desirable if consumed before exercise. Orally taken after being dissolved in a suitable amount of, if preferred, warm water, yogurt, milk, soy milk, milkshake, tea, vegetable juice, liquid coffee, almond milk, coconut juice, fermented glutinous rice soup, mineral water, or pure water (where the following drinks: juice, sugar water, and high sugar drinks are forbidden! No fruits and snacks are allowed in the conditioning process!). 1 course of treatment per month. Observation over 3 courses of treatment is needed.

### Example 6: Red bean-Chinese date meal replacement tea powder

Mixing ratio of raw materials: red bean 30 parts, Chinese date 20 parts, rose flower 10 parts, L-carnitine 10 parts, oat 10 parts, soybean protein isolate 5 parts, konjac flour 5 parts, soybean oligosaccharide 5 parts, and corn starch 5 parts.

Preparation process: the same as that in Example 5.

Applicable to: those having local obesity or obesity complicated by yellowish tongue coating, and essentially determined to be "Yang obese".

Usage and dosage: the same as that in Example 5.

### Example 7: Dedicated meal replacement tea powder of fried licorice root (spring)

Mixing ratio of raw materials: fried licorice root 35 parts, Chinese date 20 parts, gardenia 15 parts, soybean oligosaccharide 10 parts, L-carnitine 10 parts, and konjac flour 10 parts.

Preparation process: The raw materials were mixed according to the above proportions, pulverized, sterilized, and packaged, to prepare a tea having a specification of 20 g/bag x 20 bags/box.

Usage and dosage: once in the evening, 4 bags each time, dedicated for consumption in the evening and in spring. The effect is more desirable if consumed before exercise. Orally taken after being dissolved in a suitable amount of, if preferred, warm water, yogurt, milk, soy milk, milkshake, tea, vegetable juice, liquid coffee, almond milk, coconut juice, fermented glutinous rice soup, mineral water, or pure water (where the following drinks: juice, sugar water, and high sugar drinks are forbidden! No fruits and snacks are allowed in the conditioning process!). 1 course of treatment per month. Observation over 3 courses of treatment is needed.

### Example 8: Dedicated meal replacement tea powder of herba lophatheri (summer)

Mixing ratio of raw materials: herba lophatheri 30 parts, semen phaseoli 22 parts, Lycium chinense 15 parts, gardenia 15 parts, soybean oligosaccharide 6 parts, L-carnitine 6 parts, and konjac flour 6 parts.

Preparation process: the same as that in Example 7.

Usage and dosage: the same as that in Example 7. The product is consumed in summer.

### Example 9: Dedicated meal replacement tea powder of Chinese yam (autumn)

Mixing ratio of raw materials: Chinese yam 30 parts, Polygonatum sibiricum 22 parts, Lycium chinense 15 parts, gardenia 15 parts, soybean oligosaccharide 6 parts, L-carnitine 6 parts, and konjac flour 6 parts.

Preparation process: the same as that in Example 7.

Usage and dosage: the same as that in Example 7. The product is consumed in autumn.

### Example 10: Dedicated meal replacement tea powder of peach kernel (winter)

Mixing ratio of raw materials: peach kernel 35 parts, laminalia 22 parts, gardenia 15 parts, soybean oligosaccharide 10 parts, L-carnitine 10 parts, and konjac flour 10 parts.

Preparation process: the same as that in Example 7.

Usage and dosage: the same as that in Example 7. The product is consumed in winter.

In the above examples, the amount of the raw materials is expressed in parts by weight.

## Claims

1. A traditional Chinese medicine healthcare preparation for fat elimination and weight reduction, which is prepared into an orally taken preparation following a pharmaceutically conventional process or prepared into health food and food according to a processing technology of health food and food, by using rose flower, soybean protein isolate, konjac flour, soybean oligosaccharide, oat, Chinese date, and L-carnitine as active pharmaceutical ingredients.

2. The traditional Chinese medicine healthcare preparation for fat elimination and weight reduction according to claim 1, wherein the active pharmaceutical ingredients further comprise black soybean and Chinese yam, and the preparation is suitable for obese patients having high blood pressure, high blood fat, high blood glucose, and hyperuricemia.

3. The traditional Chinese medicine healthcare preparation for fat elimination and weight reduction according to claim 2, wherein the raw materials are mixed in the following parts by weight: black soybean 28-35 parts, Chinese yam 12-15 parts, Chinese date 12-15 parts, rose flower 8-10 parts, L-carnitine 8-10 parts, oat 8-10 parts, soybean protein isolate 5-6 parts, konjac flour 5-6 parts, and soybean oligosaccharide 4-6 parts.

4. The traditional Chinese medicine healthcare preparation for fat elimination and weight reduction according to claim 3, wherein the raw materials are mixed in the following parts by weight: black soybean 30 parts, Chinese yam 13 parts, Chinese date 13 parts, rose flower 10 parts, L-carnitine 10 parts, oat 10 parts, soybean protein isolate 6 parts, konjac flour 6 parts, and soybean oligosaccharide 5 parts.

5. The traditional Chinese medicine healthcare preparation for fat elimination and weight reduction according to claim 1, wherein the active pharmaceutical ingredients further comprise corn starch, mulberry leaves, and gardenia, and the preparation is suitable for obese patients caused by too much water intake.

6. The traditional Chinese medicine healthcare preparation for fat elimination and weight reduction according to claim 5, wherein the raw materials are mixed in the following parts by weight: mulberry leaves 22-28 parts, Chinese date 12-15 parts, gardenia 12-15 parts, rose flower 8-10 parts, L-carnitine 8-10 parts, oat 7-10 parts, soybean protein isolate 4-6 parts, konjac flour 4-6 parts, soybean oligosaccharide 4-6 parts, and corn starch 4-6 parts.

7. The traditional Chinese medicine healthcare preparation for fat elimination and weight reduction according to claim 6, wherein the raw materials are mixed in the following parts by weight: mulberry leaves 25 parts, Chinese date 13 parts, gardenia 13 parts, rose flower 10 parts, L-carnitine 10 parts, oat 9 parts, soybean protein isolate 5 parts, konjac flour 5 parts, soybean oligosaccharide 5 parts, and corn starch 5 parts.

8. The traditional Chinese medicine healthcare preparation for fat elimination and weight reduction according to claim 1, wherein the active pharmaceutical ingredients further comprise Lycium chinense, Polygonatum sibiricum, pearl barley, and semen phaseoli, and the preparation is suitable for obese patients caused by too much food intake.

9. The traditional Chinese medicine healthcare preparation for fat elimination and weight reduction according to claim 8, wherein the raw materials are mixed in the following parts by weight: Polygonatum sibiricum 20-25 parts, Chinese date 9-12 parts, Lycium chinense 9-12 parts, pearl barley 9-12 parts, semen phaseoli 9-12 parts, rose flower 7-8 parts, oat 7-8 parts, L-carnitine 7-8 parts, soybean protein isolate 2-4 parts, konjac flour 2-4 parts, and soybean oligosaccharide 2-4 parts.

10. The traditional Chinese medicine healthcare preparation for fat elimination and weight reduction according to claim 9, wherein the raw materials are mixed in the following parts by weight: Polygonatum sibiricum 23 parts, Chinese date 11 parts, Lycium chinense 11 parts, pearl barley 11 parts, semen phaseoli 11 parts, rose flower 8 parts, oat 8 parts, L-carnitine 8 parts, soybean protein isolate 3 parts, konjac flour 3 parts, and soybean oligosaccharide 3 parts.

11. The traditional Chinese medicine healthcare preparation for fat elimination and weight reduction according to claim 1, wherein the active pharmaceutical ingredients further comprise Lycium chinense, peach kernel, dried persimmon, laminalia, herba lophatheri, gardenia, Polygonatum sibiricum, pearl barley, and mulberry leaves, and the preparation is suitable for patients with generalized-type of obesity.

12. The traditional Chinese medicine healthcare preparation for fat elimination and weight reduction according to claim 11, wherein the raw materials are mixed in the following parts by weight: Lycium chinense 18-24 parts, Chinese date 8-12 parts, peach kernel 8-12 parts, rose flower 4-6 parts, oat 4-6 parts, L-carnitine 4-6 parts, dried persimmon 4-6 parts, laminalia 4-6 parts, herba lophatheri 4-6 parts, gardenia 4-6 parts, Polygonatum sibiricum 4-6 parts, pearl barley 4-6 parts, mulberry leaves 4-6 parts, soybean protein isolate 3-4 parts, konjac flour 2-3 parts, and soybean oligosaccharide 2-3 parts.

13. The traditional Chinese medicine healthcare preparation for fat elimination and weight reduction according to claim 12, wherein the raw materials are mixed in the following parts by weight: Lycium chinense 20 parts, Chinese date 10 parts, peach kernel 10 parts, rose flower 5 parts, oat 5 parts, L-carnitine 5 parts, dried persimmon 5 parts, laminalia 5 parts, herba lophatheri 5 parts, gardenia 5 parts, Polygonatum sibiricum 5 parts, pearl barley 5 parts, mulberry leaves 5 parts, soybean protein isolate 4 parts, konjac flour 3 parts, and soybean oligosaccharide 3 parts.

14. The traditional Chinese medicine healthcare preparation for fat elimination and weight reduction according to claim 1, wherein the active pharmaceutical ingredients further comprise pearl barley, and the preparation is suitable for "Yin-obese" patients.

15. The traditional Chinese medicine healthcare preparation for fat elimination and weight reduction according to claim 14, wherein the raw materials are mixed in the following parts by weight: pearl barley 30-35 parts, Chinese date 18-22 parts, rose flower 8-12 parts, L-carnitine 8-12 parts, oat 8-12 parts, soybean protein isolate 5-7 parts, konjac flour 5-7 parts, and soybean oligosaccharide 4-6 parts.

16. The traditional Chinese medicine healthcare preparation for fat elimination and weight reduction according to claim 15, wherein the raw materials are mixed in the following parts by weight: pearl barley 33 parts, Chinese date 20 parts, rose flower 10 parts, L-carnitine 10 parts, oat 10 parts, soybean protein isolate 6 parts, konjac flour 6 parts, and soybean oligosaccharide 5 parts.

17. The traditional Chinese medicine healthcare preparation for fat elimination and weight reduction according to claim 1, wherein the active pharmaceutical ingredients further comprise red bean and corn starch, and the preparation is suitable for "Yang-obese" patients.

18. The traditional Chinese medicine healthcare preparation for fat elimination and weight reduction according to claim 17, wherein the raw materials are mixed in the following parts by weight: red bean 28-32 parts, Chinese date 18-22 parts, rose flower 8-12 parts, L-carnitine 8-12 parts, oat 8-12 parts, soybean protein isolate 5-7 parts, konjac flour 5-7 parts, soybean oligosaccharide 5-7 parts, and corn starch 5-7 parts.

19. The traditional Chinese medicine healthcare preparation for fat elimination and weight reduction according to claim 18, wherein the raw materials are mixed in the following parts by weight: red bean 30 parts, Chinese date 20 parts, rose flower 10 parts, L-carnitine 10 parts, oat 10 parts, soybean protein isolate 5 parts, konjac flour 5 parts, soybean oligosaccharide 5 parts, and corn starch 5 parts.

20. The traditional Chinese medicine healthcare preparation for fat elimination and weight reduction according to any one of claims 1 to 19, wherein the orally taken preparation is in the form of tablets, granules, pills, tea preparations, and capsules.

21. The traditional Chinese medicine healthcare preparation for fat elimination and weight reduction according to any one of claims 1 to 19, wherein the health food and food are biscuits, meal replacement tea powder, solid beverage, coffee, and bread.

22. A seasonal traditional Chinese medicine healthcare preparation for fat elimination and weight reduction, which is prepared into an orally taken preparation following a pharmaceutically conventional process or prepared into health food and food according to a processing technology of health food and food, by using gardenia, soybean oligosaccharide, L-carnitine, konjac flour as basic active pharmaceutical ingredients.

23. A seasonal traditional Chinese medicine healthcare preparation for fat elimination and weight reduction according to claim 22, wherein the active pharmaceutical ingredients further comprise fried licorice root and Chinese date, and the preparation is suitable for fat eliminating and health preservation in spring.

24. A seasonal traditional Chinese medicine healthcare preparation for fat elimination and weight reduction according to claim 23, wherein the raw materials are mixed in the following parts by weight: fried licorice root 32-38 parts, Chinese date 19-22 parts, gardenia 14-18 parts, soybean oligosaccharide 6-10 parts, L-carnitine 6-10 parts, and konjac flour 6-10 parts.

25. A seasonal traditional Chinese medicine healthcare preparation for fat elimination and weight reduction according to claim 24, wherein the raw materials are mixed in the following parts by weight: fried licorice root 35 parts, Chinese date 20 parts, gardenia 15 parts, soybean oligosaccharide 10 parts, L-carnitine 10 parts, and konjac flour 10 parts.

26. A seasonal traditional Chinese medicine healthcare preparation for fat elimination and weight reduction according to claim 22, wherein the active pharmaceutical ingredients further comprise herba lophatheri, semen phaseoli, and Lycium chinense, and the preparation is suitable for fat eliminating and health preservation in summer.

27. A seasonal traditional Chinese medicine healthcare preparation for fat elimination and weight reduction according to claim 26, wherein the raw materials are mixed in the following parts by weight: herba lophatheri 28-32 parts, semen phaseoli 20-24 parts, Lycium chinense 14-17 parts, gardenia 14-18 parts, soybean oligosaccharide 6-10 parts, L-carnitine 6-10 parts, and konjac flour6-10 parts.

28. A seasonal traditional Chinese medicine healthcare preparation for fat elimination and weight reduction according to claim 27, wherein the raw materials are mixed in the following parts by weight: herba lophatheri 30 parts, semen phaseoli 22 parts, Lycium chinense 15 parts, gardenia 15 parts, soybean oligosaccharide 6 parts, L-carnitine 6 parts, and konjac flour 6 parts.

29. A seasonal traditional Chinese medicine healthcare preparation for fat elimination and weight reduction according to claim 22, wherein the active pharmaceutical ingredients further comprise Chinese yam, Polygonatum sibiricum, and Lycium chinense, and the preparation is suitable for fat eliminating and health preservation in autumn.

30. A seasonal traditional Chinese medicine healthcare preparation for fat elimination and weight reduction according to claim 29, wherein the raw materials are mixed in the following parts by weight: Chinese yam 28-32 parts, Polygonatum sibiricum 20-24 parts, Lycium chinense 14-17 parts, gardenia 14-18 parts, soybean oligosaccharide 6-10 parts, L-carnitine 6-10 parts, and konjac flour 6-10 parts.

31. A seasonal traditional Chinese medicine healthcare preparation for fat elimination and weight reduction according to claim 30, wherein the raw materials are mixed in the following parts by weight: Chinese yam 30 parts, Polygonatum sibiricum 22 parts, Lycium chinense 15 parts, gardenia 15 parts, soybean oligosaccharide 6 parts, L-carnitine 6 parts, and konjac flour 6 parts.

32. A seasonal traditional Chinese medicine healthcare preparation for fat elimination and weight reduction according to claim 22, wherein the active pharmaceutical ingredients further comprise peach kernel, and laminalia, and the preparation is suitable for fat eliminating and health preservation in winter.

33. A seasonal traditional Chinese medicine healthcare preparation for fat elimination and weight reduction according to claim 32, wherein the raw materials are mixed in the following parts by weight: peach kernel 33-37 parts, laminalia 20-23 parts, gardenia 14-18 parts, soybean oligosaccharide 6-10 parts, L-carnitine 6-10 parts, and konjac flour 6-10 parts.

34. A seasonal traditional Chinese medicine healthcare preparation for fat elimination and weight reduction according to claim 33, wherein the raw materials are mixed in the following parts by weight: peach kernel 35 parts, laminalia 22 parts, gardenia 15 parts, soybean oligosaccharide 10 parts, L-carnitine 10 parts, and konjac flour 10 parts.

35. A seasonal traditional Chinese medicine healthcare preparation for fat elimination and weight reduction according to claim 22, wherein the orally taken preparation is in the form of tablets, granules, pills, tea preparations, and capsules.

36. A seasonal traditional Chinese medicine healthcare preparation for fat elimination and weight reduction according to any one of claims 22 to 35, wherein the health food and food are biscuits, meal replacement tea powder, solid beverage, coffee, and bread.
